# EUROPÄISCHE PATENTANMELDUNG

(11) **EP 1 006 193 A2**
(43) Veröffentlichungstag der Anmeldung: **07.06.2000**
(21) Anmeldenummer: 99122651.5
(22) Anmeldetag: 13.11.1999
(51) Int. Cl.: C12N 15/60, C12N 15/54, C12N 15/77, C12P 13/02, C12N 1/21

(54) **Verfahren zur fermentativen Herstellung von D-Pantothensäure unter Verwendung von Stämmen der Familie Enterobacteriaceae**

(30) Priorität: 01.12.1998 DE 19855314
(71) Anmelder: Degussa-Hüls Aktiengesellschaft, 60287 Frankfurt am Main (DE)
(72) Erfinder: Rieping, Mechthild, Dr., 33619 Bielefeld (DE); Thierbach, Georg, Dr., 33613 Bielefeld (DE); Pfefferle, Walter, Dr., 33790 Halle (DE); Dusch, Nicole, Dr., 33619 Bielefeld (DE); Kalinowski, Jörn, Dr., 33615 Bielefeld (DE); Pühler, Alfred, Prof.Dr., 33739 Bielefeld (DE)

(57) **Zusammenfassung**

Die Erfindung betrifft ein Verfahren zur Herstellung von D-Pantothensäure durch Fermentation von D-Pantothensäure produzierenden Mikroorganismen der Familie Enterobacteriacae, das dadurch gekennzeichnet ist, daß man Stämme einsetzt, die
a) das Plasmid pFV31 und/oder pFV202 enthalten, und man in diesen Mikroorganismen
b) das panD-Gen und gegebenfalls weitere für die Aspartat-1-decarboxylase codierende Nukleotidsequenzen verstärkt, insbesondere überexprimiert,
c) die Pantothensäure im Medium oder in den Zellen der Mikroorganismen anreichert und
d) die gebildete Pantothensäure isoliert.

## Beschreibung

### Stand der Technik

Die Pantothensäure stellt ein kommerziell bedeutendes Vitamin dar, das in der Kosmetik, der Medizin, der Humanernährung und in der Tierernährung Anwendung findet.

Pantothensäure kann durch chemische Synthese oder biotechnisch durch Fermentation geeigneter Mikroorganismen in geeigneten Nährlösungen hergestellt werden. Bei der chemischen Synthese ist das DL-Pantolacton eine wichtige Zwischenstufe. Es wird in einem mehrstufigen Verfahren aus Formaldehyd, Isobutylaldehyd und Cyanid hergestellt. In weiteren Verfahrensschritten wird das racemische Gemisch aufgetrennt und D-Pantolacton mit β-Alanin kondensiert und man erhält D-Pantothensäure.

Der Vorteil der fermentativen Herstellung durch Mikroorganismen liegt in der direkten Bildung der gewünschten D-Form der Pantothensäure, die frei von L-Pantothensäure ist.

Verschiedene Arten von Bakterien, wie z. B. Escherichia coli, Arthrobacter ureafaciens, Corynebacterium erythrogenes, Brevibacterium ammoniagenes und auch Hefen, wie z. B. Debaromyces castellii können wie in EP-A 0 493 060 gezeigt, in einer Nährlösung, die Glucose, DL-Pantoinsäure und β-Alanin enthält, D-Pantothensäure produzieren. EP-A 0 493 060 zeigt weiterhin, daß bei Escherichia coli durch Amplifikation von Pantothensäure-Biosynthesegenen, die auf den Plasmiden pFV3 und pFV5 enthalten sind, in einer Nährlösung, die Glucose, DL-Pantoinsäure und β-Alanin enthält, die Bildung von D-Pantothensäure verbessert wird.

EP-A 0 590 857 und US-Patent 5,518,906 betreffen von Escherichia coli Stamm IFO3547 abgeleitete Mutanten wie FV5714, FV525, FV814, FV521, FV221, FV6051 und FV5069 die Resistenzen gegen verschiedene Antimetabolite wie Salizylsäure, α-Ketobuttersäure, β-Hydroxyasparaginsäure, O-Methylthreonin und α-Ketoisovaleriansäure tragen und in einer Nährlösung, die Glucose enthält, Pantoinsäure, und in einer Nährlösung, die Glucose und β-Alanin enthält, D-Pantothensäure produzieren.

In EP-A 0 590 857 wird weiterhin gezeigt, daß nach Amplifikation von nicht genau spezifizierten Pantothensäure-Biosynthesegenen, die auf dem Plasmid pFV31 enthalten sind, in den oben genannten Stämmen in einer Nährlösung, die Glucose enthält, die Produktion von D-Pantoinsäure und in einer Nährlösung, die Glucose und β-Alanin enthält, die Produktion von D-Pantothensäure verbessert wird.

In WO 97/10340 wird darüber hinaus gezeigt, daß nach Erhöhung der Aktivität des Enzyms Acetohydroxysäure-Synthase II durch Amplifikation der ilvGM-Gene mittels Plasmid pFV202, einem Enzym der Valin Biosynthese, in einer Nährlösung, die Glucose enthält, die Produktion von D-Pantoinsäure und in einer Nährlösung, die Glucose und β-Alanin enthält, die Produktion von D-Pantothensäure verbessert wird.

Den genannten Textstellen ist nicht zu entnehmen, inwieweit die genannten Stämme in einer Nährlösung, die lediglich Glucose oder lediglich Saccharose als Substrat enthalten, Pantothensäure produzieren.

### Aufgabe der Erfindung

Die Erfinder haben sich zur Aufgabe gestellt Pantothensäure-bildende Stämme der Familie Enterobacteriaceae, insbesondere der Gattung Escherichia weiter zu verbessern.

### Beschreibung der Erfindung

Das Vitamin Pantothensäure stellt ein kommerziell bedeutendes Produkt dar, das in der Kosmetik, der Medizin, der Humanernährung und in der Tierernährung Anwendung findet. Es besteht daher ein allgemeines Interesse daran, verbesserte Verfahren zur Herstellung von Pantothensäure bereitzustellen.

Wenn im Folgenden D-Pantothensäure oder Pantothensäure oder Pantothenat erwähnt werden, sind damit nicht nur die freie Säure sondern auch die Salze der D-Pantothensäure wie z.B. das Calcium-, Natrium-, Ammonium- oder Kaliumsalz gemeint.

Gegenstand der Erfindung ist ein Verfahren zur Herstellung von D-Pantothensäure durch Fermentation von D-Pantothensäure produzierenden Mikroorganismen der Familie Enterobacteriaceae,insbesondere der Gattung Escherichia, das dadurch gekennzeichnet ist, daß diese
a) das Plasmid pFV31 und/oder pFV202, bevorzugt pFV31 enthalten, und in denen man
b) das panD-Gen und gegebenfalls weitere für die Aspartat-1-decarboxylase (E.C. 4.1.1.11) codierende Nukleotidsequenzen verstärkt, bevorzugt überexprimiert.

Bevorzugt setzt man Mikroorganismen mit einer DNA ein, deren Nucleotidsequenz für pandD mit der Herkunft aus E.coli, insbesondere coryneformen Bakterien codiert. Bevorzugt handelt es sich um eine replizierbare DNA, die dadurch gekennzeichnet ist, daß
(i) die Nucleotidseguenz, gezeigt in SEQ.-ID.-Nr. 1, für panD codiert, oder
(ii) diese der Sequenz (i) innerhalb des Bereichs der Degeneration des genetischen Codes entspricht, oder
(iii) diese mit einer zur Sequenz (i) oder (ii) komplementären Sequenz hybridisiert und gegebenenfalls
(iiii) diese funktionsneutrale Sinnmutationen in (i) trägt.

Die Fermentation findet bevorzugt in einer Nährlösung statt, die ausschließlich Glucose oder Saccharose als Substrat enthält und frei von β-Alanin und Pantoinsäure ist.

Die Produktion der Pantothensäure wird so erfindungsgemäß verbessert. Der Zusatz von Aspartat, β-Alanin, Ketoisovalerat, Ketopantoinsäure oder Pantoinsäure und/oder deren Salzen wird gegebenenfalls gewünscht.

Der Begriff 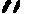 Verstärkung" beschreibt in diesem Zusammenhang die Erhöhung der intrazellulären Aktivität eines oder mehrerer Enzyme in einem Mikroorganismus, die durch die entsprechende DNA kodiert werden, indem man beispielsweise die Kopienzahl des Gens bzw. der Gene erhöht, einen starken Promotor verwendet oder ein Gen verwendet, das für ein entsprechendes Enzym mit einer hohen Aktivität kodiert und gegebenenfalls diese Maßnahmen kombiniert.

Die Mikroorganismen, die Gegenstand der vorliegenden Erfindung sind, können Pantothensäure aus Glucose, Saccharose, Lactose, Fructose, Maltose, Melasse, Stärke, Cellulose oder aus Glycerin und Ethanol herstellen. Bevorzugt werden Glucose oder Saacharose eingesetzt. Es handelt sich dabei insbesondere um Gram-negative Bakterien, wie z. B. die der Familie Enterobacteriaceae. Bei diesen ist besonders die Gattung Escherichia mit der Art Escherichia coli zu nennen. Innerhalb der Art Escherichia coli sind die sogenannten K-12 Stämme, wie z. B. die Stämme MG1655 oder W3110 (Neidhard et al.: Escherichia coli and Salmonella. Cellular and Molecular Biology (ASM Press, Washington D.C.)) oder der Escherichia coli Wildtypstamm IFO3547 (Institut für Fermentation, Osaka, Japan) und davon abgeleitete Mutanten geeignet, die die Fähigkeit besitzen Pantothensäure zu produzieren. Hierbei sind besonders die Stämme 3547/pFV31, 5714/pFV31, 525/pFV31, 814/pFV31, 521/pFV31, FV221/pFV31, FV6051/pFV31, FV5069/pFV31, FV5069/pFV202 und solche, die durch konventionelle Mutagenese, Selektion, z. B. auf Antimetabolit-Resistenz, wie Azidothymidin-Resistenz, Thiaisoleucin-Resistenz und Screening aus diesen hervorgegangen sind, zu nennen. Speziell geeignet sind die Stämme FV5069/pFV31 und FV5069/pFV202, die als FERM BP 4395 und FERM BP 5227 gemäß Budapester Vertrag hinterlegt sind (siehe EP-A-0590857 und WO 97/10340).

Zur Erzielung einer Verstärkung, insbesondere einer Überexpression, erhöht man z. B. die Kopienzahl der entsprechenden Gene, oder mutiert die Promotor- und Regulationsregion, die sich stromaufwärts des Strukturgens befindet. In gleicher Weise wirken Expressionskassetten, die stromaufwärts des Strukturgens eingebaut werden. Durch induzierbare Promotoren ist es zusätzlich möglich, die Expression im Verlaufe der fermentativen D-Pantothenatbildung zu steigern. Durch Maßnahmen zur Verlängerung der Lebensdauer der m-RNA wird ebenfalls die Expression verbessert. Weiterhin wird durch Verhinderung des Abbaus des Enzymproteins ebenfalls die Enzymaktivität verstärkt. Die Gene oder Genkonstrukte können entweder in Plasmiden mit unterschiedlicher Kopienzahl vorliegen oder im Chromosom integriert und amplifiziert sein. Alternativ kann weiterhin eine Überexpression der betreffenden Gene durch Veränderung der Medienzusammensetzung und Kulturführung erreicht werden.

Anleitungen hierzu findet der Fachmann unter anderem bei Chang und Cohen (Journal of Bacteriology 134:1141-1156 (1978)), bei Hartley und Gregori (Gene 13:347-353 (1981)), bei Amann und Brosius (Gene 40:183-190 (1985)), bei de Breer et al. (Proceedings of the National of Sciences of the United States of America 80:21-25 (1983)), bei LaVallie et al. (BIO/TECHNOLOGY 11, 187-193 (1993)), in PCT/US97/13359, bei Llosa et al. (Plasmid 26:222-224 (1991)), bei Quandt und Klipp (Gene 80:161-169 (1989)), bei Hamilton (Journal of Bacteriology 171:4617-4622 (1989), bei Jensen und Hammer (Biotechnology and Bioengineering 58, 191-195 (1998) und in bekannten Lehrbüchern der Genetik und Molekularbiologie.

Das panD-Gen von E. coli ist bekannt. Die Nukleotidsequenz wurde von Merkels und Nichols (FEMS Microbiology Letters 143, 247-252 (1996)) publiziert. Zur Isolierung bzw. Herstellung des panD-Gens von E. coli kann eine PCR- (Polymerase Kettenreaktion) Methode, wie sie allgemein bekannt ist, oder eine der unten aufgeführten Methoden angewendet werden.

Zur Isolierung des panD-Gens von C. glutamicum wird zunächst eine Genbank dieses Mikrorganismus in E. coli angelegt. Das Anlegen von Genbanken ist in allgemein bekannten Lehrbüchern und Handbüchern niedergeschrieben. Als Beispiel seien das Lehrbuch von Winnacker: Gene und Klone, Eine Einführung in die Gentechnologie (Verlag Chemie, Weinheim, Deutschland, 1990) oder das Handbuch von Sambrook et al.: Molecular Cloning, A Laboratory Manual (Cold Spring Harbor Laboratory Press, 1989) genannt. Eine bekannte Genbank ist die des E. coli K-12 Stammes W3110, die von Kohara et al. (Cell 50, 495 - 508 (1987)) die in λ-Vektoren angelegt wurde. Bathe et al. (Molecular and General Genetics, 252:255-265, 1996) beschreiben eine Genbank von C. glutamicum ATCC13032, die mit Hilfe des Cosmidvektors SuperCos I (Wahl et al., 1987, Proceedings of the National Academy of Sciences USA, 84:2160-2164) im E.coli Stamm K-12 NM554 (Raleigh et al., 1988, Nucleic Acids Research 16:1563-1575) angelegt wurde. Zur Herstellung einer Genbank von C. glutamicum in E. coli können auch Plasmide wie pBR322 (Bolivar, Life Sciences, 25, 807-818 (1979)) oder pUC9 (Vieira et al., 1982, Gene, 19:259-268) verwendet werden. Als Wirte eignen sich besonders solche E.coli-Stämme, die restriktions- und rekombinationsdefekt sind. Ein Beispiel hierfür ist der Stamm DH5αMCR, der von Grant et al. (Proceedings of the National Academy of Sciences USA, 87 (1990) 4645-4649) beschrieben wurde.

Die Genbank wird anschließend in einen Indikatorstamm durch Transformation (Hanahan, Journal of Molecular Biology 166, 557-580, 1983) oder Elektroporation (Tauch et.al., 1994, FEMS Microbiological Letters, 123:343-347) eingebracht. Der Indikatorstamm zeichnet sich dadurch aus, dass er eine Mutation in dem interessierenden Gen besitzt, die einen detektierbaren Phänotyp z.B. eine Auxotrophie hervorruft. Im Rahmen der vorliegenden Erfindung ist die E.coli Mutante DV9 (Vallari und Rock, Journal of Bacteriology 1985, 164:136-142), die eine Mutation im panD-Gen trägt, von besonderem Interesse. Nach Transformation des Indikatorstammes wie z.B. der panD-Mutante DV9 mit einem rekombinanten Plasmid, welches das interessierende Gen, wie z.B. das panD-Gen trägt und Expression desselben, wird der Indikatorstamm bezüglich der entsprechenden Eigenschaft wie z.B. der Pantothensäure-Bedürftigkeit prototroph.

Das dergestalt isolierte Gen bzw. DNA-Fragment kann durch Bestimmung der Sequenz, wie z.B. bei Sanger et al. (Proceedings of the National of Sciences of the United States of America USA, 74:5463-5467, 1977) beschrieben, charakterisiert werden.

Auf diese Weise wurde die neue, für das Gen panD kodierende DNA-Sequenz von C. glutamicum erhalten, die als SEQ ID NO 1 Bestandteil der vorliegenden Erfindung ist. Weiterhin wurden aus der vorliegenden DNA-Sequenz mit den oben beschriebenen Methoden die Aminosäuresequenz des entsprechenden Enzyms abgeleitet. In SEQ ID NO 2 ist die sich ergebende Aminosäuresequenz des panD-Genproduktes nämlich der L-Aspartat 1-Decarboxylase dargestellt.

Kodierende DNA-Sequenzen, die sich aus SEQ ID NO 1 durch die Degeneriertheit des genetischen Codes ergeben, sind ebenfalls Bestandteil der Erfindung. In gleicher Weise sind DNA-Sequenzen, die mit SEQ ID NO 1 hybridisieren Bestandteil der Erfindung. In der Fachwelt sind weiterhin konservative Aminosäureaustausche wie z.B. Austausch von Glycin gegen Alanin oder von Asparaginsäure gegen Glutaminsäure in Proteinen als 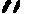 Sinnmutationen" (sense mutations) bekannt, die zu keiner grundsätzlichen Veränderung der Aktivität des Proteins führen, d.h. funktionsneutral sind. Weiterhin ist bekannt, daß Änderungen am N- und/oder C-Terminus eines Proteins dessen Funktion nicht wesentlich beeinträchtigen oder sogar stabilisieren können.

Angaben hierzu findet der Fachmann unter anderem bei Ben-Bassat et al. (Journal of Bacteriology 169:751-757 (1987)), bei O'Regan et al. (Gene 77:237-251 (1989)), bei Sahin-Toth et al. (Protein Sciences 3:240-247 (1994)), bei Hochuli et al. (Bio/Technology 6:1321-1325 (1988)) und in bekannten Lehrbüchern der Genetik und Molekularbiologie.

Das auf diese Weise isolierte und charakterisierte Gen kann anschließend einzeln oder gegebenenfalls in Kombination mit anderen in den geeigneten Stämmen von Escherichia coli zur Expression gebracht werden. Eine bekannte Methode Gene zu exprimieren bzw. überzuexprimieren besteht darin, diese mit Hilfe von Plasmidvektoren zu amplifizieren, die überdies mit Expressionssignalen ausgestattet sein können. Als Plasmidvektoren kommen solche in Frage, die in den entsprechenden Mikroorganismen replizieren können. Für E. coli kommen z.B. die Vektoren pSC101 (Vocke and Bastia, Proceedings of the National Academy of Sciences USA 80 (21), 6557-6561 (1983)) oder pKK223-3 (Brosius and Holy, Proceedings of the National Academy of Sciences USA 81, 6929 (1984)) oder der Corynebacterium glutamicum/Escherichia coli Pendelvektor pZ8-1 (Europäische Patentschrift 0 375 889) für die vorliegende Erfindung in Frage. Beispiele für derartige Stämme von E. coli sind FV5069/pFV31/pND-D1 und FV5069/pFV31 pND-D2, die die Plasmide pND-D1 und pND-D2 enthalten. Plasmid pND-D1 ist ein auf dem Plasmid pZ8-1 basierender E. coli-C. glutamicum Pendelvektor, der das panD-Gen von E. coli trägt. Plasmid pND-D2 ist ein auf dem Plasmid pZ8-1 basierender E. coli-C. glutamicum Pendelvektor der das panD-Gen von C. glutamicum trägt.

Es ist dem Fachmann klar, daß chromosomale Mutationen, die Resistenzen gegen Metabolite und Antimetabolite bewirken oder die das Abfliessen von Vorstufen der Pantothensäure verhindern,einzeln oder gemeinsam in vorteilhafter Weise mit den Massnahmen, die Gegenstand der Erfindung sind, kombiniert werden können.

Die erfindungsgemäß hergestellten Mikroorganismen können kontinuierlich oder diskontinuierlich im batch - Verfahren (Satzkultivierung) oder im fed batch (Zulaufverfahren) oder repeated fed batch Verfahren (repetitives Zulaufverfahren) zum Zwecke der Pantothensäure-Produktion kultiviert werden. Eine Zusammenfassung über bekannte Kultivierungsmethoden sind im Lehrbuch von Chmiel (Bioprozesstechnik 1. Einführung in die Bioverfahrenstechnik (Gustav Fischer Verlag, Stuttgart, 1991)) oder im Lehrbuch von Storhas (Bioreaktoren und periphere Einrichtungen (Vieweg Verlag, Braunschweig/Wiesbaden, 1994)) beschrieben.

Das zu verwendende Kulturmedium muß in geeigneter Weise den Ansprüchen der jeweiligen Mikroorganismen genügen. Beschreibungen von Kulturmedien verschiedenener Mikroorganismen sind im Handbuch "Manual of Methods for General Bacteriology" der American Society for Bacteriology (Washington D.C., USA, 1981) enthalten. Als Kohlenstoffquelle können Zucker und Kohlehydrate wie z.B. Glucose, Saccharose, Lactose, Fructose, Maltose, Melasse, Stärke und Cellulose, Öle und Fette wie z. B. Sojaöl, Sonnenblumenöl, Erdnussöl und Kokosfett, Fettsäuren wie z. B. Palmitinsäure, Stearinsäure und Linolsäure, Alkohole wie z. B. Glycerin und Ethanol und organische Säuren wie z. B. Essigsäure verwendet werden. Diese Stoffe können einzeln oder als Mischung verwendet werden. Als Stickstoffquelle können organische Stickstoff haltige Verbindungen wie Peptone, Hefeextrakt, Fleischextrakt, Malzextrakt, Maisquellwasser, Sojabohnenmehl und Harnstoff oder anorganische Verbindungen wie Ammoniumsulfat, Ammoniumchlorid, Ammoniumphosphat, Ammoniumcarbonat und Ammnoniumnitrat verwendet werden. Die Stickstoffquellen können einzeln oder als Mischung verwendet werden. Als Phosphorquelle können Kaliumdihydrogenphosphat oder Dikaliumhydrogenphosphat oder die entsprechenden Natrium haltigen Salze verwendet werden. Das Kulturmedium muß weiterhin Salze von Metallen enthalten wie z.B. Magnesiumsulfat oder Eisensulfat, die für das Wachstum notwendig sind. Schließlich können essentielle Wuchsstoffe wie Aminosäuren und Vitamine zusätzlich zu den oben genannten Stoffen eingesetzt werden. Dem Kulturmedium können, wenn es gewünscht wird, die Vorstufen der Pantothensäure wie Aspartat, β-Alanin, Ketoisovalerat oder Ketopantoinsäure oder Pantoinsäure und gegebenenfalls deren Salze zugesetzt werden. Die genannten Einsatzstoffe können zur Kultur in Form eines einmaligen Ansatzes hinzugegeben oder in geeigneter Weise während der Kultivierung zugefüttert werden.

Zur pH - Kontrolle der Kultur werden basische Verbindungen wie Natriumhydroxid, Kaliumhydroxid, Ammoniak oder saure Verbindungen wie Phosphorsäure oder Schwefelsäure in geeigneter Weise eingesetzt. Zur Kontrolle der Schaumentwicklung können Antischaummittel wie z.B. Fettsäurepolyglykolester eingesetzt werden. Zur Aufrechterhaltung der Stabilität von Plasmiden können dem Medium geeignete selektiv wirkende Stoffe z.B. Antibiotika hinzugefügt werden. Um aerobe Bedingungen aufrechtzuerhalten werden Sauerstoff oder Sauerstoff haltige Gasmischungen wie z.B. Luft in die Kultur eingetragen. Die Temperatur der Kultur liegt normalerweise bei 25°C bis 45°C und vorzugsweise bei 30°C bis 37°C. Die Kultur wird solange fortgesetzt bis sich ein Maximum an Pantothensäure gebildet hat. Dieses Ziel wird normalerweise innerhalb von 10 Stunden bis 160 Stunden erreicht.

Es ist dem Fachmann klar, daß Stämme, die eine hohe Aktivität des Enzyms L-Aspartat 1-Decarboxylase besitzen, auch für die Herstellung von β-Alanin aus L-Aspartat eingesetzt werden können. Hierzu setzt man die bekannten fermentativen Verfahren, enzymatischen Umwandlungs-reaktionen oder Kombinationen beider ein.

Die Konzentration an gebildeter Pantothensäure kann mit bekannten Verfahren (Velisek; Chromatographic Science 60, 515-560 (1992)) bestimmt werden.

Folgende Mikroorganismen wurden bei der Deutschen Sammlung für Mikroorganismen und Zellkulturen (DSMZ, Braunschweig, Deutschland) gemäß Budapester Vertrag hinterlegt:

Corynebacterium glutamicum ATCC13032/pND-D2 als DSM12438

### Beispiele

Die vorliegende Erfindung wird im folgenden anhand von Ausführungsbeispielen näher erläutert.

### Beispiel 1

### Klonierung und Sequenzierung des panD-Gens von C. glutamicum

### 1. Klonierung des panD-Gens

Chromosomale DNA aus C. glutamicum ATCC 13032 wurde wie bei Tauch et al. (1995, Plasmid, 33:168-179) beschrieben, isoliert und mit dem Restriktionsenzym Sau3A (Pharmacia Biotech (Freiburg, Deutschland), Produktbeschreibung Sau3A, Code no. 27-0913-02) partiell gespalten. DNA-Fragmente in einem Größenbereich von 7-9 kb wurden mit Hilfe des "Nucleotrap Extraction Kit for Nucleic Acids" (Macherey und Nagel, Düren, Deutschland; Cat. No. 740584) isoliert und in die dephosphorylierte BamHI-Schnittstelle des Vektors pUC19 (Norrander et al., 1982, Gene, 26:101-106), der von der Firma MBI Fermentas (Vilnius,Litauen) bezogen wurde, ligiert. Die Ligation wurde wie von Sambrook et al. (1989, Molecular Cloning: A laboratory Manual, Cold Spring Harbor) beschrieben durchgeführt, wobei das DNA-Gemisch mit T4-Ligase (Pharmacia Biotech, Freiburg, Deutschland) über Nacht inkubiert wurde. Dieses Ligationsgemisch wurde anschließend in den E. coli Stamm DH5αMCR (Grant, 1990, Proceedings of the National Acadimies of Sciences USA, 87:4645-4649) elektroporiert (Tauch, 1994, FEMS Microbiological Letters, 123:343-347) und auf LB-Agar (Lennox, 1955, Virology, 1:190) + 100 µg/ml Ampicillin ausplattiert. Nach Inkubation für 24 Stunden bei 37°C konnte die C. glutamicum Genbank durch Reisolierung der Plasmid-DNA nach der "Alkalischen-Lyse-Methode" von Birnboim und Doly (Nucleic Acids Research, 7: 1513-1523, 1997) aus den Transformanten gewonnen werden. Mit dieser Genbank wurden kompetente Zellen des E. coli Stamms DV9 (Vallari und Rock, 1985, Journal of Bacteriology, 164:136-142), welcher eine Mutation im panD-Gen trägt, elektroporiert. Der Elektroporationsansatz wurde im Anschluß an die Regenerationsphase (Tauch et.al., 1994, FEMS Microbiological Letters, 123:343-347) zweimal mit Medium E (Vogel and Bonner, 1956, Journal of Biolgical Chemistry, 218:97-106) gewaschen. Die Zusammensetzung des Mediums E ist in Tabelle 1 dargestellt. Mit diesen Zellen wurden 50 ml Medium E + 100 µg/ml Ampicillin, die in einem 250 ml Erlenmeyerkolben vorlagen, inocculiert und in einem Luftschüttler bei 250 U/min und 39°C inkubiert. Nach zweitägiger Inkubation wurde die Bakteriensuspension verdünnt und auf LB-Agar (Lennox, 1955, Virology, 1:190) ausgestrichen, der mit 100 µg/ml Ampicillin supplementiert worden war.

**Tabelle 1**

| Substanz | Menge pro Liter | Bemerkung |
|---|---|---|
| K₂HPO₄ | 10 g | |
| NaNH₄HPO₄*4 H₂O | 3,5 g | |
| Zitronensäure | 2 g | |
| MgSO₄ * 7 H₂O | 0,2 g | |
| Glukose | 4 g | separat sterilisieren |
| Thiamin | 0,2 µg | sterilfiltrieren |

Die Plasmid-DNA einer DV9-Transformante wurde isoliert, als pNIC-1.3 bezeichnet und mittels Agarosegelelektrophorese (Sambrook et al., Molecular cloning. A laboratory manual (1989) Cold Spring Harbour Laboratory Press) und Vergleich mit Standard-DNA-Fragmenten bekannter Länge charakterisiert. Plasmid pNIC-1.3 enthält eine Insertion von 7 kbp Länge. Die Komplementationsfähigkeit von pNIC-1.3 wurde durch erneute Transformation der panD Mutante DV9 überprüft.

Die erhaltenen Transformanten waren wiederum fähig, in β-Alanin-freiem Medium E unter den oben angegebenen Bedingungen zu wachsen.

Die Subklonierung des 7 kb Inserts erfolgte durch Spaltung des Plasmids pNIC-1.3 mit den Restriktionsenzymen BamHI (Pharmacia Biotech (Freiburg, Deutschland), Produktbeschreibung BamHI, Code no. 27-0868-03), EcoRI (Pharmacia Biotech (Freiburg, Deutschland), Produktbeschreibung EcoRI, Code no. 27-0884-03) und BglII (Pharmacia Biotech (Freiburg, Deutschland), Produktbeschreibung BglII, Code no. 27-0946-02) und anschließender Ligation in den entsprechend restriktionsverdauten Vektor pK18mob (Schäfer, 1994, Gene, 145:69-73). Der erhaltene Ligationsansatz wurde in die E. coli panD Mutante DV9 elektroporiert; die Selektion auf komplementierte Transformanten erfolgte wie oben beschrieben wobei die Aqarplatten in diesem Fall 50 µg/ml Kanamycin enthielten. Die Plasmide von komplementierten Einzelklonen wurden isoliert und mittels Restriktionsanalysen charakterisiert. Ein EcoRI-Subklon, im Folgenden pNIC-10 genannt, mit einem ungefähr 3 kb großen DNA-Insert wurde für die folgende Sequenzanalyse ausgewählt.

### 2. Sequenzierung des panD-Gens

Für die doppelsträngige Sequenzierung des 3 kb Fragments von pNIC-10 wurde dieses mit verschiedenen Restriktionsenzymen gespalten und die Fragmente in die Plasmide pUC19 oder pK18mob subkloniert. Die zum Sequenzieren eingesetzte Plasmid-DNA wurden nach Herstellerangaben mit dem "QIAGEN Plasmid Mini kit" (Qiagen, Inc., Chatsworth, Ca., USA) isoliert und die Bestimmung der Plasmidgrößen wurde mittels Agarosegelelektrophorese durchgeführt.

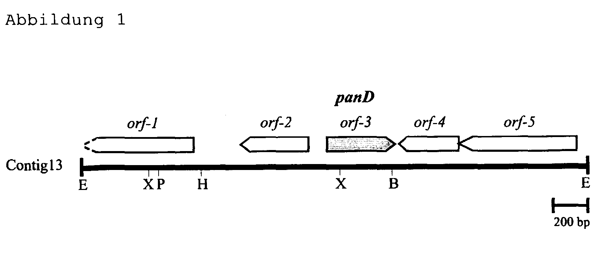

Die Sequenzierung erfolgte nach der Dideoxy-Kettenabbruch-Methode von Sanger et al. (Proceedings of the National Academies of Sciences USA, 74:5463-5467, 1977) mit Modifikationen nach Zimmermann et al. (Nucleic Acids Research, 18:1067, 1990). Es wurde der "Cy5-AutoRead Sequencing kit" von Pharmacia (Product No. 27-2690-02, Freiburg, Germany) angewandt. Die gelelektrophoretische Auftrennung und Analyse der Sequenzierreaktion erfolgte in einem "Long Ranger Gel Solution"-Polyacrylamidgel (FMC BioProducts, Rockland, Me., USA) mit dem "automatischen Laser-Fluoreszenz (A.L.F.) Express DNA Sequenziergerät" von Amersham Pharmacia Biotech (Uppsala, Schweden). Die erhaltenen Roh-Sequenzdaten wurden anschließend unter Anwendung des Staden-Programpakets (Nucleic Acids Researeh, 14:217-231, 1986) Version 97-0 prozessiert. Sämtliche Einzelsequenzen der pNIC-10 Subklone wurden zu einem zusammenhängenden 3060 bp langen Contig assembliert, der als Contig13 bezeichnet wurde. Die computergestützte Kodierbereichsanalyse mit dem Programm XNIP (Staden, 1986, Nucleic Acids Research, 14:217-231) des gesamten DNA-Fragments ergab die Identifizierung von fünf offenen Leserasten (ORFs). In Abbildung 1 ist eine Restriktionskarte von Contig13 sowie die Lage der als orf-1 bis orf-5 bezeichneten ORFs dargestellt. Homologieanalysen wurden mit den "BLAST search programs" (Gish and States, 1993, Nature of Genetics, 3:266-272; Altschul et al., 1990, Journal of Molecular Biology, 215:403-410), welche über den Online-Service des NCBI-Servers der "National Library of Medicine" (Bethesda, MD, USA) zur Verfügung gestellt wurde, durchgeführt. Die Analyse von Contig13 ergab, daß orf-3 das panD-Gen ist. Im Folgenden wird orf-3 als panD bezeichnet. Die Nukleotidsequenz des das panD-Gen tragenden DNA-Fragmentes ist als SEQ ID NO 1 wiedergegeben. Die Aminosäuresequenz des sich mit obigen Methoden ergebenden panD-Genproduktes nämlich der L-Aspartat 1-Decarboxylase ist als SEQ ID NO 2 wiedergegeben.

### Beispiel 2

### Konstruktion von Vektoren zur Expression von panD-Genen

Die Pantothenatbiosynthese-Gene panD aus C. glutamicum und panD aus E. coli wurden unter Anwendung der Polymerase-Kettenreaktion (PCR) sowie synthetischen Oligonucleotiden amplifiziert. Die PCR-Experimente wurden mit der Taq DNA polymerase der Firma Gibco-BRL (Eggestein, Deutschland) in einem "PCT-100 Thermocycler" (MJ Research Inc., Watertown, Mass., USA) durchgeführt. Auf einen einmaligen Denaturierungsschritt von 2 Minuten bei 94°C folgte ein Denaturierungsschritt von 90 Sekunden bei 94°C, ein Annealingschritt für 90 Sekunden bei einer Primer-abhängigen Temperatur von T=(2AT+4GC) - 5 °C (Suggs, et al., 1981, S. 683-693, In: D. D. Brown, and C. F. Fox (Eds.), Developmental biology using purified genes. Academic Press, New York, USA) sowie ein 90 sec dauernder Extensionsschritt bei 72°C. Die letzten drei Schritte wurden 35 mal zyklisch wiederholt und die Reaktion wurde mit einem finalen Extensionsschritt von 10 Minuten bei 72°C beendet. Die so amplifizierten Produkte wurden, nachdem sie im Agarosegel elektrophoretisch geprüft worden sind, den Herstellerangaben entsprechend in den Vektor pCR®2.1 (Original TA Clonong Kit, Invitrogene (Leek, Niederlande), Produktbeschreibung Original TA Cloning® Kit, Cat. no. KNM2030-01).) ligiert und anschließend in den E. coli Stamm TOP10F' transformiert. Die Selektion auf Transformanten erfolgte durch Inkubation bei 37 °C für 24 Stunden auf LB-Agarplatten mit 100 µg/ml Ampicillin und 40 µg/ml X-Gal (5-Bromo-4-Chloro-3-Indolyl-β-D-Galaktosid).

Ausgehend von den Nucleotidsequenzen des Pantothenatbiosynthese-Gens panD von C. glutamicum ATCC 13032 (Abbildung 2) und von E. coli K12 (W.K. Merkel and B.P. Nichols, 1993, GenBank: L17086) wurden PCR-Primer synthetisiert (MWG Biotech, Ebersberg, Deutschland). Diese Primer wurden so ausgewählt, daß die amplifizierten Fragmente die Gene sowie deren native Ribosomen-Bindestellen, nicht aber mögliche Promotor-Regionen enthalten. Zusätzlich wurden geeignete Restriktionsschnittstellen eingefügt, die das Klonieren in den Zielvektor ermöglichen. Die Sequenzen der PCR-Primer, die eingefügten Schnittstellen (Sequenz unterstrichen) sowie das amplifizierte Gen (Fragmentgröße in bp ist in Klammern angegeben) sind in der folgenden Tabelle 2 aufgelistet.

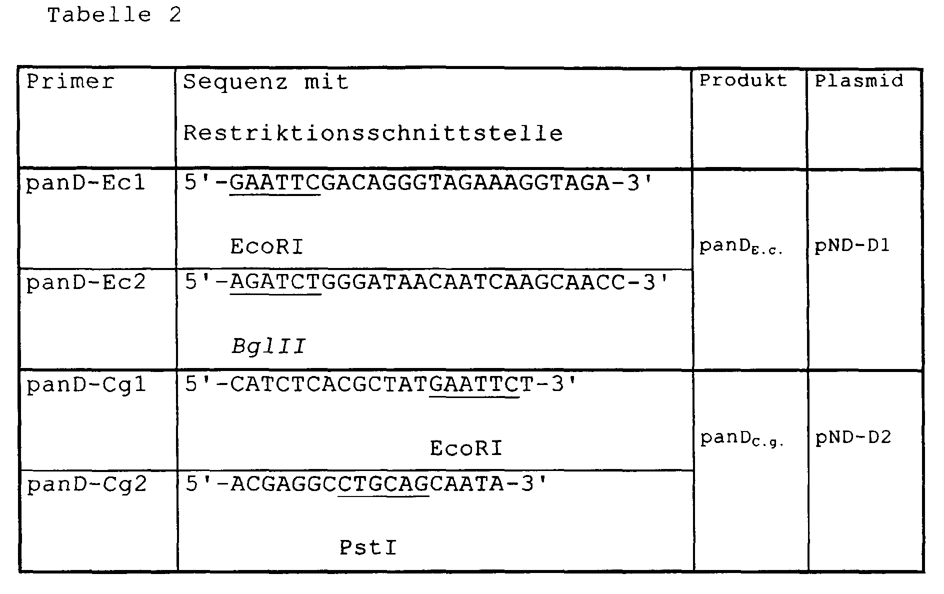

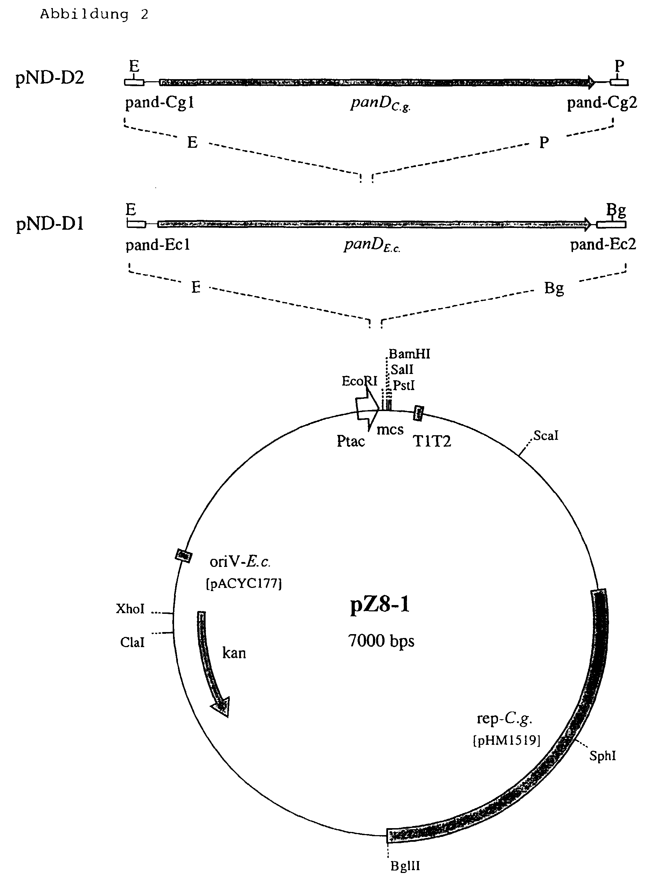

Als Basisvektor zur Expression sowohl in C. glutamicum als auch in E. coli wurde der in Abbildung 2 dargestellte E. coli-C. glutamicum-Shuttle-Expressionsvektor pZ8-1 (Europäische Patentschrift 0 375 889) eingesetzt. Das zuvor in den Vektor pCR®2.1 klonierten panD_{C.g.} Amplifikat wurden mittels der Primerinserierten Restriktionsschnittstellen in den ebenso behandelten Expressionsvektor pZ8-1 ligiert und somit unter die Kontrolle des auf diesem Plasmid enthaltenen tac-Promotors gebracht. Das Amplifikat panD_{E.c.} wurde als EcoRI-BglII-Fragment in die kompatiblen EcoRI-BamHI-Restriktionsenden des Vektors pZ8-1 kloniert. Die jeweiligen Plasmidbezeichnungen für die so konstruierten Expressionsplasmide sind in der Tabelle 2 angegeben. Die Klonierungsstrategie für die Gene panD_{E.c.} und panD_{C.g.} ist in der Abbildung 2 dargestellt. Die korrekte Klonierung der Expressionsplasmide wurde durch Sequenzierung der jeweiligen Inserts überprüft.

Diese Plasmide pND-D1 und pND-D2 wurden in den E. coli Stamm FV5069/pFV31 transformiert und Transformanten auf LB-Agar (Lennox, 1955, Virology, 1:190) + 50 µg/ml Kanamycin selektioniert. Die erhaltenen Stämme wurden FV5069/pFV31/pND-D1 und FV5069/pFV31/pND-D2 genannt.

### Beispiel 3

### Bildung von Pantothenat durch verschiedene von E. coli FV5069/pFV31 abgeleitete Stämme

Die quantitative Bestimmung von D-Pantothenat erfolgte mittels des Lactobacillus plantarum Pantothenat-Assays (Teststamm: Lactobacillus plantarum ATCC 8014, Cat. No.3211-30-3; Kulturmedium: Bacto Pantothenate Assay Medium (DIFCO Laboratories, Michigan, USA), Cat. No. 0604-15-3) Dieser Indikatorstamm kann nur bei Anwesenheit von Pantothenat im angegebenen Kulturmedium wachsen und zeigt eine photometrisch meßbare, lineare Abhängigkeit des Wachstums von der Pantothenat-Konzentration des Mediums. Für die Kalibrierung wurde das Hemicalciumsalz von Pantothenat eingesetzt (Sigma, Produktbezeichnung P 2250). Die optische Dichte wurde an einem LKB Biochrom Photometer der Firma Pharmacia Biotech (Freiburg, Deutschland) bei einer Messwellenlänge von 580 nm (o.D.₅₈₀)bestimmt.

Die Pantothenat-Produktion der E. coli Stämme FV5069/pFV31, FV5069/pFV31/pND-D1 und FV5069/pFV31/pND-D2 mit Glucose oder Saccharose als Substrat wurde bestimmt. Als Testmedium wurde Medium E verwendet, das entweder 4 g/l Glucose oder 4 g/l Saccharose als Substrat enthieltund im Falle der Stämme FV5069/pFV31/pND-D1 und FV5069/pFV31/pND-D2 mit 50 µg/ml Kanamycin supplementiert wurde. 50 ml Testmedium, die in einem 500 ml Erlenmeyerkolben vorlagen, wurden ausgehend von einer 16 Stunden alten Kultur des gleichen Mediums so angeimpft, daß die o.D.₅₈₀ von 0,1 betrug. Nach 72 Stunden Inkubation dieser Kulturen bei 37°C und 250 U/min wurden die Zellen durch 10-minütige Zentrifugation bei 5000 x g pelletiert. Der erhaltene zellfreie Überstand wurde sterilfiltriert und bis zur Pantothenat-Quantifizierung bei 4 °C gelagert.

Die Quantifizierung des D-Pantothenats im Kulturüberstand erfolgte mittels L. plantarum ATCC 8014 nach Angaben des Handbuchs der Firma DIFCO (DIFCO MANUAL, 10^{th} Edition, S. 1100-1102; Michigan, USA). Die Ergebnisse dieser Messungen sind in der Tabelle 3 und Tabelle 4 dargestellt.

**Tabelle 3**

| Pantothenat-Akkumulation mit Glucose als Substrat | | |
|---|---|---|
| Stamm | Gen | Pantothenat (µg/ml) |
| FV5069/pFV31 | - | 2 |
| FV5069/pFV31/pND-D1 | panD_{E.c}. | 24 |
| FV5069/pFV31/pND-D2 | panD_{c.g.} | 58 |

**Tabelle 4**

| Pantothenat-Akkumulation mit Saccharose als Substrat | | |
|---|---|---|
| Stamm | Gen | Pantothenat (µg/ml) |
| FV5069/pFV31 | - | 2 |
| FV5069/pFV31/pND-D1 | panD_{E.c.} | 47 |
| FV5069/pFV31/pND-D2 | panD_{C.g.} | 69 |

SEQUENZPROTOKOLL

(1) ALLGEMEINE ANGABEN:

(i) ANMELDER:
(A) NAME: Degussa Aktiengesellschaft
(B) STRASSE: Weissfrauenstr. 9
(C) ORT: Frankfurt am Main
(D) BUNDESLAND: Hessen
(E) LAND: Deutschland
(F) POSTLEITZAHL: D-60311

(ii) BEZEICHNUNG DER ERFINDUNG: Verfahren zur
fermentativen Herstellung von D-Pantothensaeure
unter Verwendung von Stämmen der Familie
Enterobacteriaceae

(iii) ANZAHL DER SEQUENZEN: 2

(iv) COMPUTER-LESBARE FASSUNG:
(A) DATENTRAEGER: Floppy disk
(B) COMPUTER: IBM PC compatible
(C) BETRIEBSSYSTEM: PC-DOS/MS-DOS
(D) SOFTWARE: PatentIn Release #1.0, Version
#1.30 (EPA)

(2) ANGABEN ZU SEQ ID NO: 1:

(i) SEQUENZKENNZEICHEN:
(A) LAENGE: 540 Basenpaare
(B) ART: Nucleotid
(C) STRANGFORM: Doppelstrang
(D) TOPOLOGIE: linear

(ii) ART DES MOLEKUELS: Genom-DNA

(iii) HYPOTHETISCH: NEIN

(iv) ANTISENSE: NEIN

(vi) URSPRÜNGLICHE HERKUNFT:
(A) ORGANISMUS: Corynebacterium glutamicum
(B) STAMM: ATCC13032

(ix) MERKMAL:
(A) NAME/SCHLUESSEL: CDS
(B) LAGE:77..484
(D) SONSTIGE ANGABEN:/codon_start= 77
/EC_number= 4.1.1.11
/product= "L-Aspartat-1-Decarboxylase"
/gene= "panD"

(xi) SEQUENZBESCHREIBUNG: SEQ ID NO: 1:

AATATTCCTT TCCTTGTCAT CTCACGCTAT GATTTCTAAA ACTTGCAGGA CAACCCCCAT 60

AAGGACACCA CAGGAC ATG CTG CGC ACC ATC CTC GGA AGT AAG ATT CAC 109
Met Leu Arg Thr Ile Leu Gly Ser Lys Ile His
1               5                   10

CGA GCC ACT GTC ACT CAA GCT GAT CTA GAT TAT GTT GGC TCT GTA ACC 157
Arg Ala Thr Val Thr Gln Ala Asp Leu Asp Tyr Val Gly Ser Val Thr
            15                  20                  25

ATC GAC GCC GAC CTG GTT CAC GCC GCC GGA TTG ATC GAA GGC GAA AAA 205
Ile Asp Ala Asp Leu Val His Ala Ala Gly Leu Ile Glu Gly Glu Lys
        30                  35                  40

GTT GCC ATC GTA GAC ATC ACC AAC GGC GCT CGT CTG GAA ACT TAT GTC 253
Val Ala Ile Val Asp Ile Thr Asn Gly Ala Arg Leu Glu Thr Tyr Val
    45                  50                  55

ATT GTG GGC GAC GCC GGA ACG GGC AAT ATT TGC ATC AAT GGT GCC GCT 301
Ile Val Gly Asp Ala Gly Thr Gly Asn Ile Cys Ile Asn Gly Ala Ala
60                  65                  70                  75

GCA CAC CTT ATT AAT CCT GGC GAT CTT GTG ATC ATC ATG AGC TAC CTT 349
Ala His Leu Ile Asn Pro Gly Asp Leu Val Ile Ile Met Ser Tyr Leu
                80                  85                  90

CAG GCA ACT GAT GCG GAA GCC AAG GCG TAT GAG CCA AAG ATT GTG CAC 397
Gln Ala Thr Asp Ala Glu Ala Lys Ala Tyr Glu Pro Lys Ile Val His
            95                  100                 105

GTG GAC GCC GAC AAC CGC ATC GTT GCG CTC GGC AAC GAT CTT GCG GAA 445
Val Asp Ala Asp Asn Arg Ile Val Ala Leu Gly Asn Asp Leu Ala Glu
        110                 115                 120

GCA CTA CCT GGA TCC GGG CTT TTG ACG TCG AGA AGC ATT TAGCGTTTTA 494
Ala Leu Pro Gly Ser Gly Leu Leu Thr Ser Arg Ser Ile
    125                 130                 135

GCTCGCCAAT ATTGCTGCCG GCCTCGTTGA AAATGGTCAT GGTGGC 540

(2) ANGABEN ZU SEQ ID NO: 2:

(i) SEQUENZKENNZEICHEN:
(A) LAENGE: 136 Aminosaeuren
(B) ART: Aminosaeure
(D) TOPOLOGIE: linear

(ii) ART DES MOLEKUELS: Protein
(xi) SEQUENZBESCHREIBUNG: SEQ ID NO: 2:

Met Leu Arg Thr Ile Leu Gly Ser Lys Ile His Arg Ala Thr Val Thr
1               5                   10                  15

Gln Ala Asp Leu Asp Tyr Val Gly Ser Val Thr Ile Asp Ala Asp Leu
            20                  25                  30

Val His Ala Ala Gly Leu Ile Glu Gly Glu Lys Val Ala Ile Val Asp
        35                  40                  45

Ile Thr Asn Gly Ala Arg Leu Glu Thr Tyr Val Ile Val Gly Asp Ala

50                  55                  60

Gly Thr Gly Asn Ile Cys Ile Asn Gly Ala Ala Ala His Leu Ile Asn
65                  70                  75                  80

Pro Gly Asp Leu Val Ile Ile Met Ser Tyr Leu Gln Ala Thr Asp Ala
                85                  90                  95

Glu Ala Lys Ala Tyr Glu Pro Lys Ile Val His Val Asp Ala Asp Asn
            100                 105                 110

Arg Ile Val Ala Leu Gly Asn Asp Leu Ala Glu Ala Leu Pro Gly Ser
        115                 120                 125

Gly Leu Leu Thr Ser Arg Ser Ile
    130                 135

### Abbildungen

Folgende Abbildungen sind beigefügt:
- Abbildung 1:: Karte des Contig13 mit orf1-orf5
- Abbildung 2:: Karte des Plasmids pZ8-1 und Klonierungs strategie der Plasmide pND-D1 und pND-D2

Die in den Abbildungen verwendeten Abkürzungen haben folgende Bedeutung:
- T1T2:: Transkriptions-Terminator des rrnB-Gens
- Ptac:: tac Promotor
- panD:: Kodierbereich des panD Gens
- rep-C.g.:: DNA-Region für Replikation in C. glutamicum
- oriV-E.c.:: Ursprung für vegetativen Transfer in E. coli
- kan:: Resistenzgen für Kanamycin
- EcoRI:: Schnittstelle des Restriktionsenzyms EcoRI
- E:: Schnittstelle des Restriktionsenzyms EcoRI
- BamHI:: Schnittstelle des Restriktionsenzyms BamHI
- B: Schnittstelle des Restriktionsenzyms BamHI
- BglII:: Schnittstelle des Restriktionsenzyms BglII
- ClaI:: Schnittstelle des Restriktionsenzyms ClaI
- H:: Schnittstelle des Restriktionsenzyms HindIII
- P:: Schnittstelle des Restriktionsenzyms PstI
- PstI:: Schnittstelle des Restriktionsenzyms PstI
- SalI:: Schnittstelle des Restriktionsenzyms SalI
- ScaI:: Schnittstelle des Restriktionsenzyms ScaI
- SphI:: Schnittstelle des Restriktionsenzyms SphI
- X:: Schnittstelle des Restriktionsenzyms XbaI
- XhoI:: Schnittstelle des Restriktionsenzyms XhoI

## Patentansprüche

1. Verfahren zur Herstellung von D-Pantothensäure durch Fermentation von D-Pantothensäure produzierenden Mikroorganismen der Familie Enterobacteriacae,
**dadurch gekennzeichnet,**
daß man Stämme einsetzt, die
a) das Plasmid pFV31 und/oder pFV202 enthalten, und man in diesen Mikroorganismen
b) ein panD-Gen und gegebenfalls weitere für die Aspartat-1-decarboxylase codierende Nukleotidsequenzen verstärkt, insbesondere überexprimiert.
c) die Pantothensäure im Medium oder in den Zellen der Mikroorganismen anreichert und
d) die gebildete Pantothensäure isoliert.

2. Verfahren gemäß Anspruch 1,
**dadurch gekennzeichnet,**
daß man Mikroorganismen mit einer replizierbaren DNA einsetzt, deren Nucleotidsequenz für panD mit der Herkunft aus einem Corynebacterium codiert.

3. Verfahren gemäß Anspruch 2,
**dadurch gekennzeichnet,**
daß die replizierbare DNA charakterisiert wird durch
(i) die Nucleotidsequenz, gezeigt in SEQ.-ID.-Nr. 1, die für panD codiert, oder
(ii) eine Sequenz, die der Sequenz (i) innerhalb des Bereichs der Degeneration des genetischen Codes entspricht; oder
(iii) eine Sequenz, die mit einer zur Sequenz (i) oder (ii) komplementären Sequenz hybridisiert und gegebenenfalls
(iiii) funktionsneutrale Sinnmutationen in (i) trägt.

4. Verfahren gemäß Anspruch 1,
**dadurch gekennzeichnet,**
daß man Mikroorganismen einsetzt, in denen man zur Erzielung der Verstärkung (Überexpressung) der Gene bzw. Nucleotidsequenzen die Kopienzahl mit Hilfe diese Gene bzw. Nucleotidsequenzen tragender Plasmidvektoren erhöht.

5. Verfahren gemäß Anspruch 1,
**dadurch gekennzeichnet,**
daß man Mikroorganismen einsetzt, in denen man zur Erzielung der Überexpression die sich stromaufwärts des Strukturgens befindende Promoter- und Regulationsregion mutiert.

6. Verfahren gemäß Anspruch 1,
**dadurch gekennzeichnet**,
daß man Mikroorganismen einsetzt, in denen man zur Erzielung der Überexpression stromaufwärts des Strukturgens Expressionskasetten einbaut.

7. Verfahren gemäß Ansruch 1,
**dadurch gekennzeichnet,**
daß man zur Erzielung der Verstärkung die Lebensdauer der mRNA, die von den oben genannten Sequenzen als Matrize abgelesen wird, verlängert und/oder den Abbau der(des) entsprechenden Enzymproteins(e) verhindert.

8. Verfahren gemäß den Ansprüchen 1 bis 7,
**dadurch gekennzeichnet**,
daß man Mikroorganismen einsetzt, die zusätzliche Metabolit- bzw. Antimetabolit-Resistenzmutationen einzeln oder gemeinsam aufweisen.

9. Verfahren gemäß Ansprüchen 1 bis 8,
**dadurch gekennzeichnet,**
daß man die Mikroorganismen zur Erzielung der Überexpression in entsprechend geänderten Kulturmedien fermentiert oder die Fermentationsführung ändert.

10. Verfahren gemäß den Ansprüchen 1 bi 9,
**dadurch gekennzeichnet,**
daß man Mikroorganismen einsetzt, in denen die Stoffwechselwege, zumindest teilweise ausgeschaltet sind, die die Pantothenat-(Pantothensäure) -bildung verringern.

11. Verfahren gemäß den Ansprüchen 1 bis 10
**dadurch gekennzeichnet,**
daß man Mikroorganismen einsetzt, in denen man zusätzlich zum panD-Gen die übrigen Gene des Stoffwechselweges der Pantothensäurebildung, insbesondere mit der Herkunft Corynebacterium, einzeln oder gemeinsam überexprimiert.

12. Verfahren gemäß Anspruch 11,
**dadurch gekennzeichnet,**
daß man Mikroorganismen einsetzt, in denen man eines oder mehrere der Gene, die für Enzyme Ketopantoat-Hydroxymethyltransferase (EC 4.1.2.12) und Pantothenat-Synthetase (EC 6.3.2.1) kodieren, zusätzlich zum panD-Gen, insbesondere mit der Herkunft Corynebacterium, überexprimiert.

13. Verfahren gemäß den Ansprüchen 11 und 12,
**dadurch gekennzeichnet,**
daß man mit verschiedenen kompatiblen, die genannten Gene einzeln enthaltenden Plasmidvektoren transformierte Mikroorganismen einsetzt.

14. Verfahren gemäß den Ansprüchen 11 und 12,
**dadurch gekennzeichnet,**
daß man einen mit einem Plasmidvektor transformierten Mikroorganismus der Art E.coli einsetzt, und der Plasmidvektor eines oder mehrere der genannten Gene einschließlich des panD-Gens trägt, in dem die genannten Gene nacheinander angeordnet sind und unter die Kontrolle eines gemeinsamen Promotors oder getrennt voneinander angeordnet unter die Kontrolle verschiedener Promotoren gestellt werden.

15. Verfahren gemäß einem oder mehreren der vorhergehenden Ansprüche,
**dadurch gekennzeichnet,**
daß man Mikroorganismen einsetzt, die den Plasmidvektor pND-D2 enthalten, der charakterisiert ist durch die in der Abb. 2 wiedergegebenen Restriktionskarte, hinterlegt als Corynebacterium glutamicum ATCC 13032/pND-D2 unter der Bezeichnung DSM 12438.

16. Verfahren zur Herstellung von Pantothensäure gemäß einem oder mehreren der vorhergehenden Ansprüche,
**dadurch gekennzeichnet,**
daß man folgende Schritte durchführt:
a) Fermentation von Mikroorganismen der Art E.coli, in denen zumindest das panD-Gen verstärkt (überexprimiert) wird, gegebenenfalls in Kombination mit dem panB- und/oder panC-Gen,
b) Anreicherung der Pantothensäure im Medium oder in den Zellen der Mikroorganismen, und
c) Isolieren der Pantothensäure.

17. Verfahren gemäß Anspruch 16,
**dadurch gekennzeichnet,**
daß die überexprimierten Gene aus Mikroorganismen der Gattung Corynebacterium stammen.

18. Verfahren gemäß den Ansprüchen 16 oder 17,
**dadurch gekennzeichnet,**
daß man in Stufe a) eine Vorstufe der Pantothensäure zusetzt, ausgewählt aus der Gruppe Aspartat, β-Alanin, Ketopantoat, Ketoisovalerat oder Pantoat.

19. Mikroorganismen der Gattung E.coli, die das Plasmid pFV 31 und/oder pFV 202 enthalten und transformiert sind durch die Einführung einer replizierbaren DNA,
charakterisiert durch,
i) die Nucleotidsequenz, gezeigt in SEQ.-ID.-Nr. 1, die für panD codiert, oder
(ii) eine Sequenz, die der Sequenz (i) innerhalb des Bereichs der Degeneration des genetischen Codes entspricht; oder
(iii) eine Sequenz, die mit einer zur Sequenz (i) oder (ii) komplementären Sequenz hybridisiert, und gegebenenfalls
(iiii) funktionsneutrale Sinnmutationen in (i).
